# EUROPEAN PATENT APPLICATION

(11) **EP 3 977 984 A1**
(43) Date of publication of application: **06.04.2022**
(21) Application number: 20812784.5
(22) Date of filing: 29.05.2020
(51) Int. Cl.: A61K 9/20, A61K 9/50, A61K 31/55, A61P 35/00

(54) **PARP INHIBITOR PELLET PREPARATION AND PREPARATION PROCESS THEREFOR**

(30) Priority: 31.05.2019 WO PCT/CN2019/089618
(71) Applicant: BeiGene, Ltd., Grand Cayman KY1-1108 (KY)
(72) Inventor: WANG, Yiping, Beijing 102206 (CN); FAN, Wenyuan, Suzhou, Jiangsu 215007 (CN); DU, Zhengming, Suzhou, Jiangsu 215007 (CN); QIU, Gang, Suzhou, Jiangsu 215007 (CN); XU, Shuo, Suzhou, Jiangsu 215007 (CN); LV, Huiru, Suzhou, Jiangsu 215007 (CN); GUO, Yuanjing, Beijing 102206 (CN)
(74) Representative: Mewburn Ellis LLP
(86) International application number: PCT/CN2020/093438
(87) International publication number: WO 2020/239097

(57) **Abstract**

The present invention relates to a PARP inhibitor pellet composition and a preparation process therefor. The pellet composition comprises a pellet and an optional additional excipient, with the pellet comprising (1) a pellet core; (2) a drug-containing layer and (3) an optional protective layer, wherein the drug-containing layer contains (a) an active ingredient and (b) a binder; when the composition comprises the protective layer, the protective layer contains (c) a coating material; and the active ingredient is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof.

## Description

### Technical Field

The present disclosure belongs to the field of pharmaceutical art, relates to a PARP inhibitor pellet formulation and a preparation method therefor, and particularly relates to a pellet formulation of (R)-2-fluoro-10a-methyl-7,8,9,10,10a,11-hexahydro-5,6,7a,11-tetraazacyclohepta[def]cyclopenta[a]fluoren-4(5H)-one and a pharmaceutically acceptable salt or hydrate thereof and a preparation method therefor.

### Background

Poly(adenosine diphosphate ribose) polymerases (Poly(ADP-Ribose) Polymerases, PARPs) are a class of proteases with important physiological functions. They are present in the nucleus of eukaryotic cells. The PARP family contains a variety of PARP enzymes, of which PARP-1 is more important. On the one hand, PARP-1 is an abundant DNA gap-sensitive protease. Once bound to a DNA gap, the molecule activates PARP to cleave NAD+ into nicotinamide and ADP-ribose and polymerize the latter to nuclear receptor proteins including histones, transcription factors and PARP itself. Adenosine diphosphate ribose multimerization plays an important role in DNA repair and genome stability. On the other hand, oxidative-stress-induced PARP over-activation consumes NAD+, which in turn results in consumption of ATP, accumulatively leading to cell dysfunction or necrosis. This intracellular suicide mechanism is implicated in the pathological mechanisms of many diseases, such as stroke, myocardial infarction, diabetes, diabetes-related cardiovascular dysfunction, shock, traumatic central nervous system injury, arthritis, enteritis, allergic encephalomyelitis and various other forms of inflammation. PARP as a target for the treatment of malignant tumors has attracted widespread attention worldwide. Olaparib is the first PARP inhibitor in the world, and this drug has been marketed in Europe and the United States.

WO 2013/097225A1 discloses poly(ADP-ribosyl)transferase (PARPs) inhibitors, and specifically discloses a compound 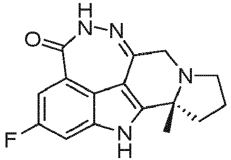 , i.e. (R)-2-fluoro-10a-methyl-7,8,9,10,10a,11-hexahydro-5,6,7a,11-tetraazacyclohepta[def]cyclopenta[a]fluoren-4(5H)-one, which compound is a poly(adenosine diphosphate (ADP)-ribose) polymerase (PARP) inhibitor that has a high selectivity for PARP-1/2 and can effectively inhibit the proliferation of cell lines with BRCA1/2 mutations or other HR defects. WO 2017/032289 A1 disclose (R)-2-fluoro-10a-methyl-7,8,9,10,10a,11-hexahydro-5,6,7a,11-tetraazacyclohepta[def]cyclopenta[a]fluoren-4(5H)-one sesquihydrate with the structure of 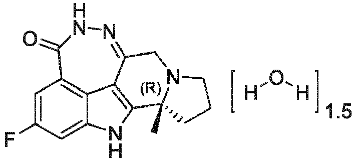 , which sesquihydrate has an excellent chemical stability and is known as Pamiparib. Pre-clinical studies have shown that Pamiparib has significant advantages in safety and effectiveness over Olaparib and other PARP inhibitors (such as Veliparib) that have entered clinical phase III by the US FDA, that is to say, it has a stronger DNA capture activity; in an experiment of a BRCA variant *in vitro* xenograft model, Pamiparib is about 16 times more active than Olaparib; and it has a better PARP1/2 selectivity, and rodents have a good tolerance to Pamiparib and a treatment window of about 10 folds; in addition, the drug has no CYP inhibitory activity and exhibits a stronger activity in combined administration and excellent pharmacokinetic properties, that is, it has excellent DMPK properties and a significant brain permeability.

However, Pamiparib has a poor fluidity, and is difficult to be directly filled and produced during the production of a formulation. Therefore, it is necessary to develop a formulation that overcomes the poor fluidity of Pamiparib and is suitable for mass production.

### Summary of the Invention

In order to overcome the deficiencies in the physical and chemical properties of the drug substance of Pamiparib in the preparation of a formulation, the inventors have made a large number of attempts in the development of a Pamiparib formulation and found that the development of Pamiparib into a pellet formulation has successfully reduced the difficulty in the formulation of the drug substance and improved the fluidity and stability of the product, thereby making large-scale commercial production possible, and facilitating transportation and storage; in addition, the preparation process is simple and convenient, no special requirements are required for the equipment, and the finally obtained finished product has a good stability, so that the present invention is suitable for large-scale production. In addition, the inventors have surprisingly discovered that mixing the prepared pellet with a certain amount of a lubricant such as talc can effectively reduce the electrostatic interaction between pellets, thereby enabling the industrial production of a pellet formulation.

Therefore, the inventors of the present invention have succeeded in improving the fluidity of the powder of the drug substance after preparing Pamiparib into pellets, and after mixing the pellets with a lubricant such as talc, the electrostatic interaction between the pellets is prevented, which is conducive to encapsulation into capsules.

On this basis, the inventors have also discovered through a large number of innovative experiments that the D₉₀ of the drug substance Pamiparib has a certain impact on the quality attributes of the final product, and as an unexpected surprise, when the D₉₀ is less than 30 µm, a final product with ideal quality attributes can be obtained.

The present invention relates to a PARP inhibitor pellet composition and a preparation method therefor; a formulation prepared using the pellet composition; and the use of the pellet composition and the formulation for treating/preventing a PARP-associated disease or condition.

In a first aspect, the present invention relates to a PARP inhibitor pellet composition, comprising a pellet and an optional additional excipient, with the pellet comprising (1) a pellet core; (2) a drug-containing layer and (3) an optional protective layer, wherein the drug-containing layer contains (a) an active ingredient and (b) a binder; when the composition comprises the protective layer, the protective layer contains (c) a coating material; and the active ingredient is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof.

In some embodiments, the present invention relates to a PARP inhibitor pellet composition, comprising a pellet and an optional additional excipient, with the pellet comprising (1) a pellet core; (2) a drug-containing layer containing (a) an active ingredient and (b) a binder; and (3) an optional protective layer, wherein the active ingredient is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof.

In some embodiments, the present invention relates to a PARP inhibitor pellet composition, comprising a pellet and an optional additional excipient, with the pellet comprising (1) a pellet core; (2) a drug-containing layer; and (3) an optional protective layer, wherein the drug-containing layer containing (a) an active ingredient and (b) a binder; the protective layer contains (c) a coating material; and the active ingredient is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof.

Preferably, the pellet is made up of, sequentially from inside to outside, (1) a pellet core, (2) a drug-containing layer; and (3) an optional protective layer.

In the above-mentioned pellet composition, the optional additional excipient includes, but is not limited to, a filler, a lubricant and other conventionally used excipients. Preferably, the additional excipient includes one or more of a filler and a lubricant, and more preferably the additional excipient includes a lubricant.

Preferably, the additional excipient is mixed with a pellet comprising (1) a pellet core; (2) a drug-containing layer; and (3) an optional protective layer.

In the above-mentioned pellet composition, the pellet core is a blank pellet core selected from one or more of a sucrose pellet core, a microcrystalline cellulose pellet core, and a starch pellet core.

In the above-mentioned pellet composition, the weight percentage of the pellet core based on the total weight of the pellet composition is 50-90%, preferably 60-85% (w/w).

In the above-mentioned pellet composition, the active ingredient is preferably crystal forms A-L of (R)-2-fluoro-10a-methyl-7,8,9,10,10a,11-hexahydro-5,6,7a,11-tetraazacyclohepta[def]cyclopenta[a]fluoren-4(5H)-one or a hydrate of (R)-2-fluoro-10a-methyl-7,8,9,10,10a,11-hexahydro-5,6,7a,11-tetraazacycloheptatrieno[def]cyclopenta[a]fluorene-4(5H)-one.

Preferably, the active ingredient is crystal form C of (R)-2-fluoro-10a-methyl-7,8,9,10,10a,11-hexahydro-5,6,7a,11-tetraazacyclohepta[def]cyclopenta[a]fluoren-4(5H)-one.

The crystal forms A-L may be prepared with reference to WO 2017/032289 A1.

Preferably, the active ingredient is a sesquihydrate of (R)-2-fluoro-10a-methyl-7,8,9,10,10a,11-hexahydro-5,6,7a,11-tetraazacyclohepta[def]cyclopenta[a]fluoren-4(5H)-one, which has the following structure:

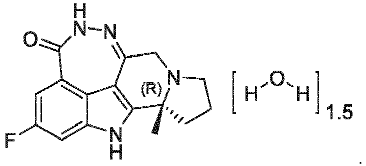

As an additional part of the present invention, the inventors have discovered that the D₉₀ of the active ingredient Pamiparib has an impact on the quality attributes of the final product.

Preferably, the D₉₀ of the active ingredient is less than 100 µm, and preferably, the D₉₀ is less than 50 µm.

As an unexpected surprise, when the D₉₀ is less than 30 µm, the final product will have an ideal final product content (99% or more), and therefore, most preferably, the D₉₀ of the active ingredient is less than 30 µm.

Preferably, the weight percentage of the active ingredient based on the total weight of the pellet composition is 5-50%, preferably 10-25%, more preferably 10-20% (w/w).

Preferably, the active ingredient is crystal form C of (R)-2-fluoro-10a-methyl-7,8,9,10,10a,11-hexahydro-5,6,7a,11-tetraazacyclohepta[def]cyclopenta[a]fluoren-4(5H)-one, the D₉₀ particle size is less than 30 µm, and the weight percentage of the active ingredient based on the total weight of the pellet composition is 10-25% (w/w), more preferably 10-20%.

Preferably, the active ingredient is a sesquihydrate of (R)-2-fluoro-10a-methyl-7,8,9,10,10a,11-hexahydro-5,6,7a,11-tetraazacyclohepta[def]cyclopenta[a]fluoren-4(5H)-one, the D₉₀ particle size is less than 30 µm, and the weight percentage of the active ingredient based on the total weight of the pellet composition is 10-25% (w/w), more preferably 10-20%.

In the above-mentioned pellet composition, the binder includes, but is not limited to, one or more of carbomer, sodium carboxymethyl cellulose, hydroxypropylcellulose, hydroxypropyl methylcellulose, and povidone.

In the above-mentioned pellet composition, the weight percentage of the binder based on the total weight of the pellet composition is 1-20%, preferably 1-10%, more preferably 3-8%, most preferably 3-6% (w/w).

Preferably, the binder is selected from hydroxypropyl methylcellulose, sodium hydroxypropyl methylcellulose and povidone.

More preferably, the binder is 3-8% (w/w) of hydroxypropyl methylcellulose and sodium hydroxypropyl methylcellulose, with the weight percentage being based on the total weight of the pellet composition.

In the above-mentioned pellet composition, the coating material includes, but is not limited to, one or more of carbomer, sodium carboxymethyl cellulose, hydroxypropylcellulose, hydroxypropyl methylcellulose, and povidone.

In the above-mentioned pellet composition, the weight percentage of the coating material based on the total weight of the pellet composition is 1-25%, preferably 1-10%, more preferably 1.5-8%, most preferably 3-6% (w/w).

Preferably, the coating material is selected from hydroxypropyl methylcellulose and sodium hydroxypropyl methylcellulose.

More preferably, the coating material is 1.5-8% (w/w) of hydroxypropyl methylcellulose and sodium hydroxypropyl methylcellulose, with the weight percentage being based on the total weight of the pellet composition.

In the above-mentioned pellet composition, the lubricant includes, but is not limited to, one or more of calcium stearate, magnesium stearate, zinc stearate, stearic acid, sodium stearyl fumarate, and talc.

In the above-mentioned pellet composition, the weight percentage of the lubricant based on the total weight of the pellet composition is 0.1-5.0%, preferably 0.1-2%, more preferably 0.5-1.5% (w/w).

After preparing Pamiparib into pellets, the pellets successfully improved the fluidity of the powder of the drug substance, which fluidity is sufficient to meet the preparation requirements, without the need for an additional lubricant to improve the fluidity of the material. In addition, the inventors have surprisingly discovered that electrostatic interaction occurs between the pellets, which has a certain impact on the filling of capsules. In order to avoid the occurrence of the static electricity problem, the inventors have surprisingly discovered that mixing a certain lubricant, especially talc, into the pellets can effectively reduce the electrostatic interaction in the pellets, making the mass commercial production of a formulation possible. Therefore, preferably, the lubricant is selected from talc.

Preferably, the lubricant is selected from 0.1-2% of talc, with the weight percentage being based on the total weight of the pellet composition.

In a second aspect, the present invention further relates to a PARP inhibitor pellet composition, comprising (1) an active ingredient that is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof; (2) a pellet core; (3) a binder; (4) an optional coating material; and (5) an optional additional excipient.

In some embodiments, the present invention relates to a PARP inhibitor pellet composition, comprising (1) an active ingredient that is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof; (2) a pellet core; (3) a binder; and (4) an optional additional excipient.

In some further embodiments, the present invention relates to a PARP inhibitor pellet composition, comprising (1) an active ingredient that is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof; (2) a pellet core; (3) a binder; (4) a coating material; and (5) an optional additional excipient.

In the above-mentioned pellet composition, the types, contents and characteristics of the active ingredient, pellet core, binder, coating material and additional excipient are as defined above.

In a third aspect, the present invention relates to a method for preparing a pellet composition. The method for preparing a pellet composition comprises the steps of:
1) dispersing an active ingredient in a binder solution to prepare a drug-containing suspension;
2) spraying the drug-containing suspension in step 1) onto the surface of a pellet core to form a drug-containing layer to prepare a drug-loaded pellet;
3) preparing a coating material solution, and spraying the coating material solution onto the surface of the drug-loaded pellet as a protective layer to prepare a protective layer pellet, this step being optionally performed; and
4) mixing the pellet obtained in step 2) or step 3) with an additional excipient to prepare a total mixture of pellet, this step being optionally performed.

In some embodiments, the present invention relates to a method for preparing a pellet composition, the method comprising the steps of:
1) dispersing an active ingredient in a binder solution to prepare a drug-containing suspension;
2) spraying the drug-containing suspension in step 1) onto the surface of a pellet core to form a drug-containing layer to prepare a drug-loaded pellet;
3) preparing a coating material solution, and spraying the coating material solution onto the surface of the drug-loaded pellet as a protective layer to prepare a protective layer pellet, thereby obtaining the pellet composition.

In some embodiments, the present invention relates to a method for preparing a pellet composition, the method comprising the steps of:
1) dispersing an active ingredient in a binder solution to prepare a drug-containing suspension;
2) spraying the drug-containing suspension in step 1) onto the surface of a pellet core to form a drug-containing layer to prepare a drug-loaded pellet;
3) mixing the pellet obtained in step 2) with an additional excipient to prepare a total mixture of pellet, thereby obtaining the pellet composition.

In some embodiments, the present invention relates to a method for preparing a pellet composition, the method comprising the steps of:
1) dispersing an active ingredient in a binder solution to prepare a drug-containing suspension;
2) spraying the drug-containing suspension in step 1) onto the surface of a pellet core to form a drug-containing layer to prepare a drug-loaded pellet;
3) preparing a coating material solution, and spraying the coating material solution onto the surface of the drug-loaded pellet as a protective layer to prepare a protective layer pellet; and
4) mixing the pellet obtained in step 3) with an additional excipient to prepare a total mixture of pellet, thereby obtaining the pellet composition.

The method of the present invention further comprises encapsulating the overall hybrid pellet into a capsule.

In the above-mentioned pellet composition, the types, contents and characteristics of the active ingredient, pellet core, binder, coating material and additional excipient are as defined above.

In a fourth aspect, the present invention relates to a PARP inhibitor oral formulation, which is prepared from the above-mentioned pellet composition.

The PARP inhibitor oral formulation is prepared from the above-mentioned pellet composition, and the oral formulation is a tablet, a capsule, or a granule, preferably a capsule.

When the oral formulation is a capsule, the capsule comprises a capsule shell. The capsule shell is selected from a gelatin hollow capsule shell and a hydroxypropyl methylcellulose hollow capsule shell, preferably a gelatin hollow capsule shell.

When the oral formulation is a capsule, different sizes of capsules can be filled according to the content of the drug substance in the pellet and the weight of the pellet, and the size includes, but is not limited to, instances in which each capsule contains 5 mg, 10 mg, 20 mg, 30 mg, 40 mg, 50 mg, 60 mg, 70 mg, 80 mg, and 100 mg of the active ingredient on the basis of the weight of (R)-2-fluoro-10a-methyl-7,8,9,10,10a,11-hexahydro-5,6,7a,11-tetraazacyclohepta[def]cyclopenta[a]fluoren-4(5H)-one.

In a fifth aspect, the present invention relates to a method for treating and/or preventing a PARP-associated disease, the method using the pellet composition of the present invention or an oral formulation prepared from the pellet composition.

The present invention further relates to the use of the pellet composition or the oral formulation prepared from the pellet composition in the preparation of a medicament for treating and/or preventing a PARP-associated disease in a mammal.

The PARP-associated disease in the present invention includes, but is not limited to, tumor angiogenesis; chronic inflammatory diseases, such as rheumatoid arthritis, atherosclerosis; dermatosis, such as psoriasis and scleroderma; diabetes-induced dermatosis, diabetic retinopathy, retinopathy of prematurity, age-related degenerative macula, cancer, hemangioma, glioma, Kaposi's sarcoma, ovarian cancer, breast cancer; lung cancers, including small cell lung cancer; pancreatic cancer, lymphoma, prostatic cancer, colon cancer and dermatoma, and complications thereof.

Among the mammals mentioned in the present application, human is preferred.

The disease is preferably selected from BRCA1 and BRCA2 mutant tumors, such as BRCA1 and BRCA2 mutant breast cancer, ovarian cancer and complications thereof.

The above-mentioned method for preventing or treating a disease may also be used in combination with any chemotherapy (for example, temozolomide (TMZ) and docetaxel), biological therapy or radiation therapy.

### Technical Terminology

Unless otherwise defined, the technical and scientific terms used in the present invention have the same meanings as commonly understood by those skilled in the art.

The singular forms "a/an" and "the" as used in the present invention include plural references.

The terms "comprise", "include" or grammatical variants thereof as used in the present invention indicate that the composition, method etc. include the listed elements and do not exclude others.

The composition of the present invention comprises a mixture of the active ingredient and other chemical ingredients.

The term optionally (optional) in the present invention indicates being possibly selected or not selected, for example, an optional additional excipient indicates containing or not containing the additional excipient.

The lubricant of the present invention includes conventionally used lubricants and/or conventionally used glidants.

The present invention provides a PARP inhibitor pellet composition, a preparation process therefor and an oral formulation (such as a capsule) prepared by using the pellet composition. In the method, the preparation of the active ingredient of (R)-2-fluoro-10a-methyl-7,8,9,10,10a,11-hexahydro-5,6,7a,11-tetraazacyclohepta[def]cyclopenta[a]fluoren-4(5H)-one into a drug-loaded pellet has successfully improved the deficiencies in terms of the physical and chemical properties of the drug substance, and improved the fluidity and stability of the product, thereby making mass commercial production possible, and facilitating transportation and storage; in addition, the preparation process is simple and convenient, and the stabilities of the final product and intermediate product are good. In addition, the intermediate pellet product has a high drug loading, and different dosages can be adjusted according to clinical indications, for the sake of convenience for patients to take.

Mixing a certain amount of a lubricant, such as talc, into the pellets can effectively reduce the electrostatic interaction in the pellets, making the mass commercial production of a formulation possible.

When the D₉₀ is less than 30 µm, the final product will be a final product provided with ideal quality attributes.

### Brief Description of the Drawings

Figure 1 is an electron microscope image of the drug substance Pamiparib.
Figure 2 is an electron microscope image of the pellet of Example 1.

### Detailed Description of Embodiments

The following examples can help those skilled in the art to understand the present invention more comprehensively, but do not limit the present invention in any way. All raw materials are commercially available.

### Example 1

Formula of a 100 g pellet formulation:

| | |
|---|---|
| Microcrystalline cellulose pellet core | 80.50 g |
| Drug-containing layer: | Pamiparib 12.08 g; and povidone 4.02 g |
| Protective layer: | hydroxypropyl methylcellulose 2.90 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluorene-4(5H)-one.

Preparation process:
1) A formula amount (4.02 g) of povidone was weighed to prepare a binder solution with a concentration of 5%, and 12.08 g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (2.90 g) of the coating material hydroxypropyl methylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

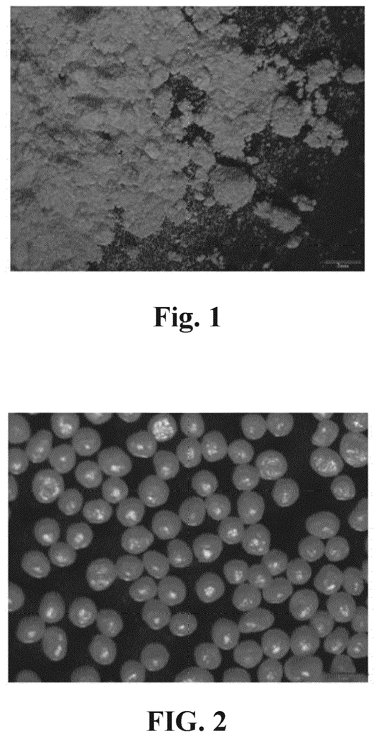

Figure 1 of the description is an electron microscope image of the drug substance Pamiparib, and since the drug substance BGB290 contains crystal water, is very easily agglomerated, and has a poor fluidity, which is not conducive to capsule filling, thereby affecting the industrialized mass production of a formulation. In addition, Figure 2 of the description is an electron microscope image of the pellets of Example 1. It can be seen from the image that the pellets are round in shape, can be evenly spread under the field of view of an electron microscope, and has a good fluidity. It is sufficient to fulfil the filling of the capsule.

### Example 2

Formula of a 100 g pellet formulation:

| | |
|---|---|
| Sucrose pellet core | 77.28 g |
| Drug-containing layer: | Pamiparib 11.60 g; and |
| hydroxypropyl methylcellulose | 7.73 g |
| Protective layer: | povidone 2.90 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (7.73 g) of hydroxypropyl methylcellulose was weighed to prepare a binder solution with a concentration of 5%, and 11.60 g of Pamiparib was uniformly dispersed in the binder solution to prepare a drug-containing layer coating suspension.
2) A formula amount of the sucrose pellet core was taken, and the drug-containing layer coating suspension was sprayed onto the surface of the pellet core to form a drug-containing layer so as to prepare a drug-loaded pellet. A formula amount (2.90 g) of the coating material povidone was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 3

Formula of a 100 g pellet formulation:

| | |
|---|---|
| Microcrystalline cellulose pellet core | 80.50 g |
| Drug-containing layer: Pamiparib 12.08 g; and hydroxypropyl methylcellulose 4.02 g | |
| Protective layer: | hydroxypropyl methylcellulose 2.90 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (4.02 g) of hydroxypropyl methylcellulose was weighed to prepare a binder solution with a concentration of 5%, and 12.08 g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (2.90 g) of the coating material hydroxypropyl methylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 4

Formula of a 100 g pellet formulation:

| | |
|---|---|
| Microcrystalline cellulose pellet core | 79.91 g |
| Drug-containing layer: | Pamiparib 12.13 g; |
| sodium carboxymethylcellulose | 4.04 g |
| Protective layer: | sodium carboxymethylcellulose 2.42 g |
| Talc | 1.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (4.04 g) of sodium carboxymethylcellulose was weighed to prepare a binder solution with a concentration of 5%, and 12.13 g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (2.42 g) of the coating material sodium carboxymethylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 5

Formula of a 100 g pellet formulation:

| | |
|---|---|
| Sucrose pellet core | 80.50 g |
| Drug-containing layer: | Pamiparib 12.08 g; |
| sodium carboxymethylcellulose | 4.02 g |
| Protective layer: | carbomer 2.90 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (4.02g) of sodium carboxymethylcellulose was weighed to prepare a binder solution with a concentration of 5%, and 12.08g of Pamiparib was uniformly dispersed in the binder solution to prepare a drug-containing layer coating suspension.
2) A formula amount of the sucrose pellet core was taken, and the drug-containing layer coating suspension was sprayed onto the surface of the pellet core to form a drug-containing layer so as to prepare a drug-loaded pellet. A formula amount (2.90 g) of the coating material carbomer was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 6

Formula of a 100 g pellet formulation:

| | |
|---|---|
| Microcrystalline cellulose pellet core | 68.43 g |
| Drug-containing layer: | Pamiparib20.53 g; and povidone6.84 g |
| Protective layer: | sodium carboxymethylcellulose 3.70 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (6.84 g) of povidone was weighed to prepare a binder solution with a concentration of 5%, and 20.53 g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (3.70g) of the coating material sodium carboxymethylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 7

Formula of a 100 g pellet formulation:

| | |
|---|---|
| Microcrystalline cellulose pellet core | 89.40 g |
| Drug-containing layer: | Pamiparib5.16 g; hydroxypropylcellulose 1.72 g |
| Protective layer: | hydroxypropyl methylcellulose 3.22 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (1.72 g) of hydroxypropylcellulose was weighed to prepare a binder solution with a concentration of 5%, and 5.16 g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (3.22g) of the coating material hydroxypropyl methylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 8

Formula of a 100 g pellet formulation:

| | |
|---|---|
| Sucrose pellet core | 81.30 g |
| Drug-containing layer: | Pamiparib 12.19 g; and hydroxypropyl methylcellulose 4.06 g |
| Protective layer: | sodium carboxymethylcellulose 1.95 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (4.06g) of hydroxypropyl methylcellulose was weighed to prepare a binder solution with a concentration of 5%, and 12.19g of Pamiparib was uniformly dispersed in the binder solution to prepare a drug-containing layer coating suspension.
2) A formula amount of the sucrose pellet core was taken, and the drug-containing layer coating suspension was sprayed onto the surface of the pellet core to form a drug-containing layer so as to prepare a drug-loaded pellet. A formula amount (1.95g) of the coating material sodium carboxymethylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 9

Formula of a 100 g pellet formulation:

| | |
|---|---|
| Microcrystalline cellulose pellet core | 78.97 g |
| Drug-containing layer: | Pamiparib 11.85 g; and povidone3.95 g |
| Protective layer: | hydroxypropyl methylcellulose 4.74 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

Preparation process:
1) A formula amount (3.95g) of povidone was weighed to prepare a binder solution with a concentration of 5%, and 11.85g of Pamiparib was uniformly dispersed in the binder solution to prepare a drug-containing layer coating suspension.
2) A formula amount of the microcrystalline cellulose pellet core was taken, and the drug-containing layer coating suspension was sprayed onto the surface of the pellet core to form a drug-containing layer so as to prepare a drug-loaded pellet. A formula amount (4.74g) of the coating material hydroxypropyl methylcellulose was taken to prepare a coating material solution with a concentration of 5%, and the coating material solution was sprayed onto the surface of the drug-loaded pellet as a protective layer to prepare a drug-loaded pellet comprising the protective layer.
3) The drug-loaded pellet (comprising the protective layer) obtained in the above-mentioned step was mixed with a formula amount of talc to prepare a total mixture of pellet.
4) The overall hybrid pellet was filled into a capsule.

Different sizes of capsules could be filled according to the content of the drug substance in the pellet and the weight of the pellet, and the size included, but was not limited to, instances in which each capsule contains 5 mg, 10 mg, 20 mg, 30 mg, 40 mg, 50 mg, 60 mg, 70 mg, 80 mg or 100 mg of the active ingredient on the basis of the anhydrous compound.

### Example 10 - Effect of the particle size of the drug substance on the content of the active ingredient in the Pamiparib pellet capsule

The inventors surprisingly discovered during the formulation development process that the D₉₀ value of the drug substance Pamiparib had a certain impact on the content of the active ingredient in the final product of the pellet capsule formulation. The same prescription as in Example 3 was used:

| Formula of a 100 g pellet formulation | |
|---|---|
| Microcrystalline cellulose pellet core | 80.50 g |
| Drug-containing layer: | Pamiparib 12.08 g; and povidone4.02 g |
| Protective layer: | hydroxypropyl methylcellulose 2.90 g |
| Talc | 0.50 g |

wherein Pamiparib was based on the total weight of the sesquihydrate of (R)-2-fluoro-10a-methyl-7,8,9,10,10a,11-hexahydro-5,6,7a,11-tetraazacyclohepta[def]cyclopenta[a]fluoren-4(5H)-one.

| | |
|---|---|
| Experimental group 1: | Pamiparib D₉₀ = 7.87 µm |
| Experimental group 2: | Pamiparib D₉₀ = 21.9 µm |
| Experimental group 3: | Pamiparib D₉₀ = 35.6 µm |
| Experimental group 4: | Pamiparib D₉₀ = 45.5 µm |

According to the method of Example 3, capsules with a content of 20 mg were prepared. The D90 was determined by using Malvern Laser Particle Sizer 3000 and using a laser diffraction method.

Determination of content: 225 mg of the capsule content pellets was weighed (allowable weighing range: 158-292 mg), the content pellets was diluted 250 times with a diluent and uniformly mixed, 3 ml was discarded using a 0.45 µm PTFE syringe filter, and the filtrate was collected and detected at a wavelength of 297 nm using a UV method or determined using HPLC. The analysis content results were as follows.

**Table 1. Content results of products prepared from the drug substance with different particle sizes**

| Experimental group | Content % |
|---|---|
| Experimental group 1 (D₉₀ = 7.87 µm) | 99.2% |
| Experimental group 2 (D₉₀ = 21.9 µm) | 99.9% |
| Experimental group 3 (D₉₀ = 35.6 µm) | 91.5% |
| Experimental group 4 (D₉₀ = 45.5 µm) | 90.6% |

It could be seen from the experimental results that when the D₉₀ was less than 30 µm, the final product had a higher content result.

The present invention has been described in detail above with the general descriptions, detailed description of embodiments and examples. Modifications or improvements based on not departing from the spirit of the present invention all fall within the scope of protection of the present invention.

## Claims

1. A PARP inhibitor pellet composition, comprising a pellet and an optional additional excipient, with the pellet comprising (1) a pellet core; (2) a drug-containing layer and (3) an optional protective layer, wherein the drug-containing layer contains (a) an active ingredient and (b) a binder; when the composition comprises the protective layer, the protective layer contains (c) a coating material; and the active ingredient is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof.

2. A PARP inhibitor pellet composition, comprising (1) an active ingredient that is (R)-2-fluoro-10a-methyl-7,8,9,10,10a,11-hexahydro-5,6,7a,11-tetraazacyclohepta[def]cyclopenta[a]fluoren-4(5H)-one, a pharmaceutically acceptable salt thereof and a hydrate thereof; (2) a pellet core; (3) a binder; (4) an optional coating material; and (5) an optional additional excipient.

3. The pellet composition according to claim 1 or 2, wherein the additional excipient includes one or more of a filler and a lubricant, and more preferably the additional excipient includes a lubricant.

4. The pellet composition according to claim 1 or 2, wherein the pellet core is a blank pellet core selected from one or more of a sucrose pellet core, a microcrystalline cellulose pellet core, and a starch pellet core; and/or
the weight percentage of the pellet core based on the total weight of the pellet composition is 50-90%, preferably 60-85% (w/w).

5. The pellet composition according to claim 1 or 2, wherein the active ingredient is crystal forms A-L or a hydrate; preferably, the active ingredient is crystal form C; preferably, the active ingredient is a sesquihydrate with the following structure:

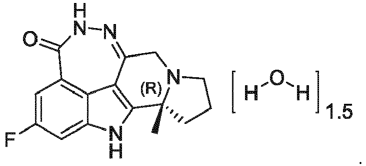

6. The pellet composition according to claim 5, wherein the active ingredient has a D₉₀ particle size of less than 100 µm, preferably a D₉₀ particle size of less than 50 µm, more preferably less than 30 µm; and/or the weight percentage of the active ingredient based on the total weight of the pellet composition is 5-50%, preferably 10-25%, more preferably 10-20% (w/w).

7. The pellet composition according to claim 1 or 2, wherein the active ingredient is crystal form C and/or a sesquihydrate of (R)-2-fluoro-10a-methyl-7,8,9,10,10a,11-hexahydro-5,6,7a,11-tetraazacyclohepta[def]cyclopenta[a]fluoren-4(5H)-one, and has a D₉₀ particle size of less than 30 µm, and the weight percentage of the active ingredient based on the total weight of the pellet composition is 10-25%.

8. The pellet composition according to claim 1 or 2, wherein the binder is selected from one or more of carbomer, sodium carboxymethyl cellulose, hydroxypropylcellulose, hydroxypropyl methylcellulose, sodium hydroxypropyl methylcellulose, and povidone; preferably, the binder is selected from hydroxypropyl methylcellulose, sodium hydroxypropyl methylcellulose, and povidone; and/or the weight percentage of the binder based on the total weight of the pellet composition is 1-20%, preferably 1-10%, more preferably 3-8%, most preferably 3-6% (w/w).

9. The pellet composition according to claim 1 or 2, wherein the coating material is selected from one or more of carbomer, sodium carboxymethyl cellulose, hydroxypropylcellulose, hydroxypropyl methylcellulose, sodium hydroxypropyl methylcellulose, and povidone; preferably, hydroxypropyl methylcellulose and sodium hydroxypropyl methylcellulose; and/or the weight percentage of the coating material based on the total weight of the pellet composition is 1-25%, preferably 1-10%, more preferably 1.5-8%, most preferably 3-6% (w/w).

10. The pellet composition according to claim 3, wherein the lubricant includes, but is not limited to, one or more of calcium stearate, magnesium stearate, zinc stearate, stearic acid, sodium stearyl fumarate, and talc, preferably talc; and/or the weight percentage of the lubricant based on the total weight of the pellet composition is 0.1-5.0%, preferably 0.1-2%, more preferably 0.5-1.5% (w/w).

11. A method for preparing the pellet composition according to any one of claims 1 and 2, comprising the steps of:
1) dispersing an active ingredient in a binder solution to prepare a drug-containing suspension;
2) spraying the drug-containing suspension in step 1) onto the surface of a pellet core to form a drug-containing layer to prepare a drug-loaded pellet;
3) preparing a coating material solution, and spraying the coating material solution onto the surface of the drug-loaded pellet as a protective layer to prepare a protective layer pellet, this step being optionally performed; and
4) mixing the pellet obtained in step 2) or step 3) with an additional excipient to prepare a total mixture of pellet, this step being optionally performed.

12. A PARP inhibitor oral formulation, wherein the PARP inhibitor oral formulation is prepared from the pellet composition according to any one of preceding claims 1-10, and the oral formulation is a tablet, a capsule, or a granule, preferably a capsule.

13. The oral formulation according to claim 12, wherein the capsule comprises a capsule shell; the capsule shell is selected from a gelatin hollow capsule shell, a hydroxypropyl methylcellulose hollow capsule shell, preferably a gelatin hollow capsule shell; and/or different sizes of capsules are filled according to the content of the active ingredient in the pellet and the weight of the pellet, and the size is selected such that each capsule contains 5 mg, 10 mg, 20 mg, 30 mg, 40 mg, 50 mg, 60 mg, 70 mg, 80 mg, or 100 mg of the active ingredient on the basis of the weight of (R)-2-fluoro-10a-methyl-7,8,9,10,10a,1 1-hexahydro-5,6,7a,1 1-tetraazacyclohepta[def]cyclopenta[a]fluoren-4(5H)-one.

14. A method for treating a PARP-associated disease, comprising administering to a patient a therapeutically effective amount of the pellet composition according to any one of claims 1-11 or the oral formulation according to any one of claims 12 and 13.

15. The method according to claim 14, wherein the PARP-associated disease is selected from tumor angiogenesis, chronic inflammatory disease, rheumatoid arthritis, atherosclerosis, dermatosis, psoriasis and scleroderma, diabetes-induced dermatosis, diabetic retinopathy, retinopathy of prematurity, age-related degenerative macula, cancer, hemangioma, glioma, Kaposi's sarcoma, ovarian cancer, breast cancer; lung cancer, small cell lung cancer, pancreatic cancer, lymphoma, prostatic cancer, colon cancer and dermatoma, and complications thereof.
